# FASCICULE DE BREVET EUROPEEN

(11) **EP 3 055 008 B1**
(45) Date de publication et mention de la délivrance du brevet: **18.07.2018**
(21) Numéro de dépôt: 14821239.2
(22) Date de dépôt: 06.10.2014
(51) Int. Cl.: A61M 5/32, A61M 5/31, A61M 5/00

(54) **DISPOSITIF DE PROTECTION POUR AIGUILLE DE SERINGUE.**
SCHÜTZENDE VORRICHTUNG FÜR EINE SPRITZENNADEL
PROTECTIVE DEVICE FOR A SYRINGE NEEDLE

(30) Priorité: 08.10.2013 FR 1359754
(43) Date de publication de la demande: 17.08.2016
(73) Titulaire: Aptar Stelmi SAS, 93420 Villepinte (FR)
(72) Inventeur: FOURNIER, Ghislain, F-17000 La Rochelle (FR); SWAL, Mickaël, F-77124 Chauconin Neufmontiers (FR)
(74) Mandataire: CAPRI
(86) Numéro de dépôt international: PCT/FR2014/052521
(87) Numéro de publication internationale: WO 2015/052417

(56) Documents cités:
- EP-A1- 1 099 450
- EP-A1- 1 208 861
- EP-A2- 0 592 814
- EP-A2- 0 976 415
- FR-A1- 2 913 200

## Description

La présente invention concerne un dispositif de protection pour aiguille de seringue comportant un capuchon élastique d'aiguille.

Plus précisément, la présente invention concerne un dispositif de protection pour aiguille de seringue du type qui comporte un capuchon élastique d'aiguille de direction générale longitudinale possédant une extrémité distale fermée et une extrémité proximale ouverte, ledit capuchon étant formé d'une paroi latérale, s'étendant depuis ladite extrémité proximale le long d'une zone d'extrémité proximale en délimitant un logement interne destiné à recevoir la portion distale du corps d'une seringue à aiguille, et d'une paroi d'extrémité dont l'épaisseur s'étend depuis ladite extrémité distale le long d'une zone d'extrémité distale, ladite paroi d'extrémité étant susceptible d'être percée sur une partie de son épaisseur par l'extrémité libre de ladite aiguille, le logement comprenant, depuis ladite extrémité proximale, une ouverture présentant un diamètre maximal, un premier tronçon de forme tronconique ou cylindrique de section circulaire, un deuxième tronçon cylindrique de section circulaire présentant un diamètre inférieur au diamètre maximal et destiné à loger la portion distale du corps de seringue qui porte ladite aiguille, et un troisième tronçon qui va en se rétrécissant depuis le deuxième tronçon jusqu'au fond dudit logement.

Des dispositifs de protection pour aiguille de seringue du type précité ont déjà été proposés.

Ainsi, le brevet EP 0 429 052 concerne un ensemble de blindage d'aiguille comprenant une gaine élastique d'aiguille similaire au capuchon élastique d'aiguille défini précédemment.

Toutefois, ce type de dispositif de protection pour aiguille présente un certain nombre d'inconvénients.

Le dispositif de protection pour aiguille de seringue objet de la présente invention est destiné à être monté sur une seringue hypodermique permettant l'injection d'un liquide médicamenteux chez un patient.

Que ces seringues hypodermiques soient préremplies ou bien qu'elles soient remplies par le personnel hospitalier juste avant la réalisation de l'injection, il est nécessaire que cette seringue reste stérile jusqu'à son utilisation.

Par exemple, lorsque la seringue est conditionnée en étant déjà remplie de liquide, sont réalisées les différentes étapes suivantes pour la préparation de la seringue avant son conditionnement. En premier lieu, le corps de la seringue, sur la partie distale duquel est montée une aiguille enduite d'un revêtement de silicone, est lavé. Ensuite, un capuchon élastique d'aiguille est monté sur l'aiguille pour former un ensemble qui sera ultérieurement rendu stérile, de préférence par un passage en autoclave.

Après le passage en autoclave de l'ensemble précité, la seringue est remplie du liquide qui lui est destiné et le corps de seringue est refermé par le piston et le poussoir qui complètent la seringue avant son conditionnement ultérieur.

Pendant le passage en autoclave, du fait que le capuchon élastique d'aiguille est réalisé dans une matière permettant le passage des gaz (généralement du caoutchouc), il est possible de réaliser un équilibre de pression entre l'extérieur du capuchon élastique, c'est-à-dire l'enceinte de l'autoclave, et l'intérieur du capuchon élastique, c'est-à-dire le logement recevant l'aiguille de la seringue.

Pendant le cycle de stérilisation en autoclave, outre une augmentation de la température dans l'enceinte de l'autoclave, on réalise également de manière classique, après un ou plusieurs vides partiels dans l'enceinte, une augmentation importante de la pression (par exemple jusqu'à 2,3 bars). Cette pression maximale est maintenue durant un certain temps (plateau de pression) avant la réalisation d'un nouveau vide partiel dans l'enceinte de l'autoclave. A la fin du cycle, la pression est augmentée jusqu'à la pression atmosphérique, tandis que la température redescend progressivement jusqu'à la température ambiante.

Des explications précédentes, on comprend que pendant le cycle de stérilisation en autoclave, il existe des changements de pression assez brutaux dans l'enceinte. Ainsi, lors de la chute brutale de la pression dans l'enceinte entre la valeur maximale de pression et le vide partiel, il arrive que la pression dans le logement du capuchon élastique ne puisse pas s'équilibrer de manière assez rapide de sorte qu'il en résulte momentanément une pression résiduelle dans ce logement qui présente une valeur supérieure à celle de la pression régnant dans l'enceinte.

Dans certains cas, notamment lorsque la paroi latérale du capuchon élastique n'est pas suffisamment résistante, la pression résiduelle présente dans le logement engendre une déformation de cette paroi latérale qui peut conduire au déplacement relatif du capuchon élastique par rapport à la portion distale du corps de seringue sur laquelle est monté le capuchon. Ce déplacement du capuchon peut même être si important qu'il peut conduire à une séparation entre le capuchon et le corps de seringue lorsque la déformation subie par le capuchon ne permet pas de retenir la portion distale du corps de seringue dans le logement.

Dans le cas du déplacement entre le capuchon et le corps de seringue, il peut exister une rupture d'étanchéité entre le logement du capuchon et l'environnement extérieur au capuchon, ce qui induit un risque de perte de stérilité dans ce logement, donc de l'aiguille. La perte de stérilité devient avérée dans le cas de la séparation entre le capuchon et le corps de seringue.

Ce déplacement induit aussi un risque de fuite de liquide hors de la seringue d'où une dose de liquide dans la seringue dont le volume a diminué et un risque de contact avec ce liquide pour l'utilisateur, ce qui peut s'avérer dangereux dans le cas de certains liquide utilisés pour des examens, notamment en imagerie médicale. Egalement, la mise en contact de l'extrémité libre de l'aiguille avec l'environnement extérieur peut engendrer des réactions physico-chimiques sur le liquide, telles que cristallisation ou coagulation, susceptibles de dégrader ce liquide et de rendre la seringue inutilisable.

Ce risque de perte de stérilité est encore plus important dans le cas où le corps de seringue est réalisé en verre, car l'utilisation de ce matériau entraîne une plage de tolérance dimensionnelle beaucoup plus étendue que dans le cas d'une seringue réalisée en matière plastique.

Le document EP 1 208 861 a proposé une solution technique comportant un bourrelet pourvu de fentes, qui a permis de sensiblement améliorer le maintien de l'aiguille dans une atmosphère stérile fermée d'une manière étanche jusqu'à son utilisation. Néanmoins, il demeure un risque de gonflement du dispositif de protection lors de la stérilisation, ce qui peut entrainer une diminution de la surface de maintien du dispositif de protection sur la seringue, avec donc un risque de séparation et de perte de stérilité lors d'une surpression à l'intérieur du dispositif de protection. De plus, lors d'une dépression dans ledit dispositif de protection, il existe un risque de rétractation de celui-ci également susceptible de rompre la stérilité.

Le document EP 0 976 415 décrit un autre dispositif de l'état de la technique.

La présente invention a pour objectif de fournir un dispositif de protection pour aiguille de seringue ne présentant pas les inconvénients précités, c'est-à-dire garantissant le maintien de l'aiguille dans une atmosphère stérile fermée d'une manière étanche jusqu'à son utilisation, en particulier lors de la phase de stérilisation. La présente invention a donc pour but d'améliorer le dispositif du document EP 1 208 861, qui divulgue le préambule de la revendication 1. La présente invention a donc pour objet un dispositif de protection pour aiguille de seringue selon la revendication 1, comportant un capuchon élastique d'aiguille de direction générale longitudinale possédant une extrémité distale fermée et une extrémité proximale ouverte, ledit capuchon comportant une paroi latérale, s'étendant depuis ladite extrémité proximale le long d'une zone d'extrémité proximale en délimitant un logement interne destiné à recevoir la portion distale du corps d'une seringue à aiguille, et d'une paroi d'extrémité dont l'épaisseur s'étend depuis ladite extrémité distale le long d'une zone d'extrémité distale, ladite paroi d'extrémité étant susceptible d'être percée sur une partie de son épaisseur par l'extrémité libre de ladite aiguille, ledit logement comprenant, depuis ladite extrémité proximale, une ouverture présentant un diamètre maximal, un premier tronçon de forme tronconique ou cylindrique de section circulaire, un deuxième tronçon cylindrique de section circulaire présentant un diamètre inférieur audit diamètre maximal et destiné à loger la portion distale du corps de seringue qui porte ladite aiguille, un troisième tronçon qui va en se rétrécissant depuis ledit deuxième tronçon en direction du fond dudit logement, ladite paroi latérale étant en outre munie d'un bourrelet annulaire disposé dans ledit logement entre lesdits premier et deuxième tronçons dudit logement, au moins une fente s'étendant en direction longitudinale sur ledit bourrelet annulaire, dans lequel ledit deuxième tronçon comporte au moins une rainure axiale rectiligne s'étendant longitudinalement sur une partie de la hauteur dudit deuxième tronçon.

Avantageusement, une pluralité, notamment quatre, fentes régulièrement angulairement réparties s'étendent en direction longitudinale sur ledit bourrelet annulaire, et une pluralité, notamment quatre, rainures régulièrement angulairement réparties s'étendent en direction longitudinale sur ledit deuxième tronçon.

Avantageusement, lesdites fentes sont disposées en quinconce avec lesdites rainures.

Avantageusement, ladite au moins une rainure a une largeur comprise entre 0,1 et 0,5 mm et/ou une profondeur d'au moins 0,1 mm et/ou une longueur inférieure d'environ 0,5 mm à la longueur dudit deuxième tronçon, avantageusement une longueur comprise entre 1,50 et 1,80 mm, de préférence environ 1,65 mm.

Avantageusement, ledit troisième tronçon comporte une projection radialement saillante, de préférence périphérique, adaptée à coopérer avec ladite partie tronconique intermédiaire de la seringue en cas de dépression dans le logement interne du capuchon pour éviter une rétractation dudit capuchon sur ladite seringue.

Avantageusement, ladite projection radialement saillante a une largeur radiale comprise entre 0,1 et 0,5 mm.

Avantageusement, le dispositif comporte en outre une coque rigide de forme générale longitudinale et cylindrique de section circulaire présentant une extrémité proximale ouverte et une extrémité distale au moins partiellement fermée, ladite coque comprenant une paroi longitudinale s'étendant depuis ladite extrémité proximale jusqu'à ladite extrémité distale et une paroi terminale située à son extrémité distale, ladite coque étant destinée à entourer en le contenant ledit capuchon élastique d'aiguille et étant pourvue de moyens de retenue dudit capuchon.

Avantageusement, lesdits moyens de retenue comportent un rebord annulaire rentrant formant l'extrémité libre proximale de la coque et présentant un diamètre intérieur apte à retenir dans la coque l'extrémité proximale de la paroi latérale du capuchon.

Avantageusement, l'extrémité proximale de la surface extérieure de la paroi latérale du capuchon comporte un épaulement annulaire rentrant en direction de l'extrémité proximale du dispositif et apte à coopérer avec ledit rebord annulaire.

Avantageusement, lesdits moyens de retenue comportent en outre une nervure annulaire disposée sur la face interne de la paroi longitudinale de la coque et apte à coopérer avec un premier épaulement rentrant en direction de l'extrémité distale du dispositif et situé sur la surface extérieure de la paroi latérale du capuchon en regard du deuxième tronçon dudit logement.

Ces caractéristiques et avantages et d'autres de la présente invention apparaîtront plus clairement au cours de la description détaillée suivante, faite en référence aux dessins joints, donnés à titre d'exemples non-limitatifs, et sur lesquels :
la figure 1 est une vue schématique en section transversale d'un dispositif de protection d'aiguille selon un mode de réalisation avantageux de la présente invention, illustrant à droite un dispositif de l'état de la technique et à gauche un dispositif selon la présente invention,
la figure 2 est une vue schématique en perspective découpée, illustrant le dispositif de protection selon le mode de réalisation de l'invention représenté sur la figure 1,
la figure 3 est une vue similaire à celle de la figure 2, avec le dispositif de protection d'aiguille assemblé sur une seringue, autour d'une aiguille,
la figure 4 est une vue similaire à celle de la figure 2, illustrant schématiquement le passage de gaz lors de la stérilisation,
la figure 5 est une vue schématique illustrant le blocage mécanique en cas de surpression dans la cavité du dispositif de protection selon la présente invention,
la figure 6 est une vue similaire à celle de la figure 5, montrant le blocage mécanique en cas de dépression dans la cavité du dispositif de protection selon la présente invention,
la figure 7 est une vue schématique illustrant le gonflement limité avec le dispositif de la présente invention, et
la figure 8 est une similaire à celle de la figure 7, illustrant un gonflement supérieur avec un dispositif selon l'état de la technique.

Dans la description qui suit de la présente invention, l'adjectif "distal" se rapporte à la partie la plus éloignée de la main de la personne tenant la seringue, et l'adjectif "proximal" se rapporte à la partie la plus proche de la main de la personne tenant la seringue.

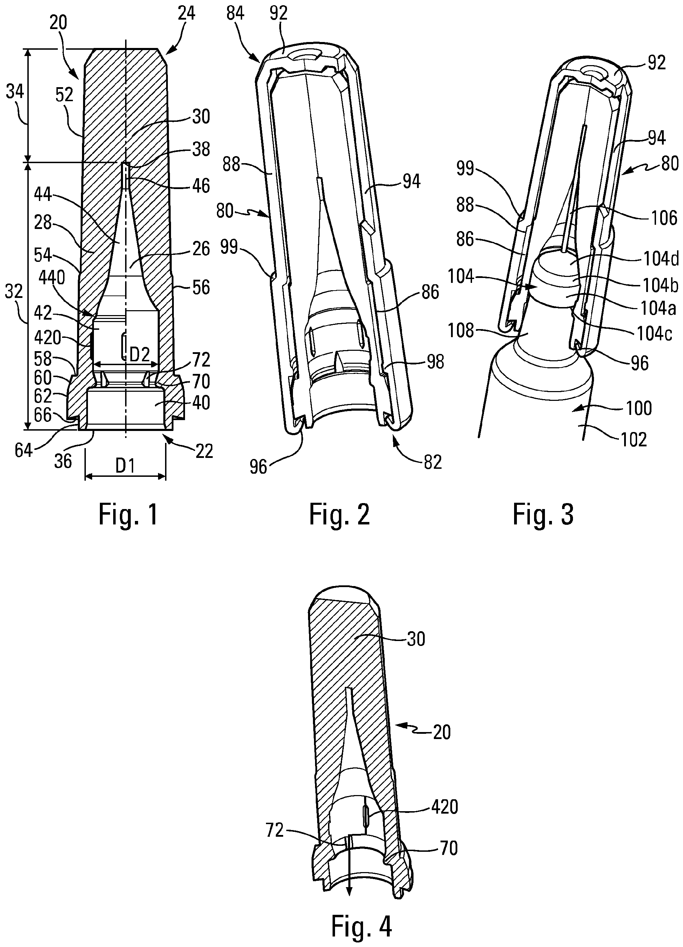

Sur la figure 1 est représenté un dispositif de protection pour aiguille de seringue composé d'un capuchon élastique d'aiguille 20 s'étendant selon une direction longitudinale entre une extrémité proximale 22 ouverte et une extrémité distale 24 fermée. Le capuchon 20 définit un logement intérieur 26 délimité par une paroi latérale 28 et par une paroi d'extrémité 30.

Il est à noter que la partie droite de la figure 1 illustre l'état de la technique selon le document EP 1 208 861, alors que la partie gauche de la figure 1 illustre la présente invention. On constatera qu'il n'y a pas de différences dans la forme extérieure du capuchon 20, les différences n'étant présentes qu'à l'intérieur de celui-ci.

La paroi latérale 28 s'étend, en direction longitudinale selon l'axe central longitudinal, depuis l'extrémité proximale 22 le long d'une zone d'extrémité proximale 32 représentant avantageusement environ les deux tiers de la longueur du capuchon 20.

La paroi d'extrémité 30 est donc pleine et s'étend, en direction longitudinale, depuis l'extrémité distale 24 le long d'une zone d'extrémité distale 34 représentant avantageusement environ un tiers de la longueur totale du capuchon 20. Ainsi, l'épaisseur de la paroi d'extrémité 30 (qui correspond à la longueur de la zone d'extrémité distale 34) permet le logement de l'extrémité libre de l'aiguille d'une seringue comme il sera expliqué ci-après.

Comme il est habituellement répandu pour ce type de dispositif de protection pour aiguille, le capuchon élastique 20 est de révolution autour de son axe central longitudinal. Cette symétrie de révolution concerne le contour externe du capuchon 20 (paroi latérale 28 et paroi d'extrémité 30) ainsi que le contour interne de la paroi latérale 28 qui définit le logement 26, sauf en ce qui concerne la présence de fentes et de rainures comme il sera expliqué ci-après.

Le logement interne 26 est composé de plusieurs tronçons s'étendant depuis une ouverture 36 située à l'extrémité proximale 22 du capuchon 20, jusqu'à un fond 38 situé de l'autre côté de la zone d'extrémité proximale 32.

De manière adjacente à l'ouverture 36, le logement 26 comporte un premier tronçon 40 qui présente une forme cylindrique de section circulaire sur les figures, une forme tronconique allant en se rétrécissant en direction du fond 38 pouvant toutefois également être utilisée.

Tel qu'illustré, le premier tronçon 40 présente un diamètre sensiblement égal au diamètre D1 de l'ouverture 36.

De l'autre côté de l'ouverture 36, le premier tronçon précité 40 est voisin d'un deuxième tronçon 42 destiné à recevoir la portion distale d'un corps de seringue qui porte l'aiguille, également appelé boule de la seringue, ce deuxième tronçon présentant une forme cylindrique de section circulaire et présentant un diamètre D2 inférieur au diamètre D1 de l'ouverture 36.

En direction opposée au premier tronçon 40, le deuxième tronçon 42 est prolongé par un troisième tronçon 44 dont le diamètre va en se rétrécissant progressivement en direction du fond 38 du logement 26. Au niveau du fond 38 du logement 26, est constitué un quatrième tronçon 46 cylindrique de section circulaire présentant un diamètre sensiblement égal au diamètre de l'aiguille de seringue.

Le contour extérieur du capuchon 20 présente une forme générale cylindrique de section circulaire avec des variations de diamètre et de forme telles qu'exposées ci-après.

A partir de l'extrémité distale 24, au niveau de laquelle la face de la paroi d'extrémité 30 tournée vers l'extérieur est sensiblement plane, s'étend une première portion 50 en forme de tronc de cône d'étendue relativement limitée, une deuxième portion 52 de forme cylindrique de section circulaire légèrement évasée qui s'étend sensiblement jusqu'à la moitié de la zone d'extrémité proximale 32 à la hauteur du troisième tronçon 44 du logement 26.

La forme légèrement évasée de la deuxième portion 52 permet de manière classique de faciliter le démoulage du capuchon et la forme de la première portion 50 facilite le centrage et le montage de la coque rigide sur le capuchon comme il sera expliqué plus loin.

Un épaulement saillant 54 relie la deuxième portion 52 à une troisième portion 56 de forme cylindrique de section circulaire très légèrement évasée qui s'étend jusqu'au deuxième tronçon 42 du logement 26 à proximité du premier tronçon 40.

Un autre épaulement saillant 58 relie la troisième portion 56 à une quatrième portion 60 de forme tronconique qui s'étend jusqu'à la hauteur du premier tronçon 40 du logement 26. Cette quatrième portion 60 est prolongée en direction de l'extrémité proximale du capuchon 20 par une cinquième portion 62 de forme cylindrique, elle-même adjacente à une sixième portion 64 située en retrait par rapport à la cinquième portion 62. Cette sixième portion 64 comporte une surface d'épaulement 66 formant un angle très légèrement inférieur à 90° par rapport à la cinquième portion 62 et s'étend vers l'ouverture 36. Ainsi, cette sixième portion 64 comprend un épaulement annulaire 66 rentrant en direction de l'extrémité proximale 22.

Entre les premier et deuxième tronçons 40 et 42 du logement 26, est prévu un bourrelet annulaire 70 formant un renflement interne de matière au niveau de l'extrémité du deuxième tronçon 42 tournée en direction de l'extrémité proximale 22.

De façon avantageuse, ce bourrelet 70 présente, en section longitudinale une forme de demi goutte d'eau dont la partie la plus épaisse est tournée en direction de l'extrémité proximale 22 du capuchon 20, la pointe de la goutte d'eau rejoignant le deuxième tronçon 42 en regard de l' autre épaulement saillant 58.

Ce bourrelet 70 délimite un contour intérieur du logement en forme de demi-poire et constitue un cordon de retenue mécanique pour la portion distale du corps de la seringue comme il sera expliqué ci-après. Lors du passage en autoclave, le bourrelet 70 améliore la retenue de la portion distale 104 de la seringue 100 dans le logement 26.

Afin de faciliter, lors du passage en autoclave, le passage de la vapeur d'eau sous pression hors du logement 26 et d'améliorer la déformabilité de ce bourrelet annulaire 70, celui-ci est muni d'une pluralité de fentes 72, avantageusement quatre, s'étendant en direction longitudinale sur le bourrelet 70, ces fentes étant régulièrement angulairement réparties.

On peut bien entendu prévoir un nombre quelconque de fentes 72, lesquelles peuvent présenter une profondeur plus ou moins importante. Si elles sont en grand nombre, ces fentes 72 séparent entre eux un grand nombre de portions du bourrelet 70 qui forment chacun une petite protubérance. De même, ces fentes 72 peuvent être plus ou moins larges.

Comme on peut le voir notamment sur la figure 3 représentant partiellement une seringue 100 hypodermique, le corps 102 cylindrique de cette seringue est muni d'une portion distale 104 qui porte l'aiguille 106.

Cette portion distale 104 présente une forme générale de sphère en constituant une boule portant l'aiguille 106. Plus précisément, la portion distale 104 forme un renflement annulaire formé de quatre parties : une partie tronconique terminale 104d allant en se rétrécissant en direction de l'extrémité libre de l'aiguille 106, une partie tronconique intermédiaire 104b formant un angle avec la partie tronconique terminale 104d, une partie médiane 104a en forme de cylindre de section circulaire et une partie tronconique de liaison 104c. La partie tronconique de liaison 104c est adjacente à une partie de révolution 108 en formant une dépression annulaire 110 par rapport à la partie la plus large de la portion distale 104.

Comme illustré sur la figure 3, lorsque l'aiguille 106 pénètre à l'intérieur du capuchon 20, l'extrémité libre de l'aiguille 106 vient se planter dans la paroi d'extrémité 30 du capuchon 20 tandis que la portion distale 104 du corps 102 de la seringue pénètre dans le logement 26 du capuchon 20 au niveau du deuxième tronçon 42.

Comme on peut le voir sur la figure 3, après montage, le bourrelet annulaire 70 est situé contre la partie tronconique de liaison 104c, ce qui réalise une retenue mécanique efficace de la partie distale de la seringue 100, formée de l'aiguille 106 et du corps cylindrique 102, à l'intérieur du logement 26.

Du fait de la présence de ce bourrelet annulaire 70 qui vient s'appliquer en contact de manière serrée contre la partie tronconique de liaison 104c, il n'est pas nécessaire que le diamètre D2 du deuxième tronçon 42 du logement 26 assure un serrage important autour de la partie médiane 104a.

Toutefois, un contact étanche doit être assuré par le deuxième tronçon 42 du logement 26 contre la partie médiane 104a de la portion distale 104 de la seringue afin de conserver stérile le logement 26 après le passage en autoclave. L'étanchéité dont il est question ici est une étanchéité microbiologique permettant de conserver une stérilité, c'est-à-dire de garantir l'absence de germes microbiens ou de produits toxiques d'origine microbienne ou fongique.

Avantageusement, le diamètre D2 du deuxième tronçon 42 du logement 26 est supérieur ou égal à 85 %, de préférence sensiblement égal à 92 %, du diamètre extérieur de la portion distale 104 du corps de seringue 102. Avantageusement également, le diamètre interne minimal du bourrelet annulaire 70 est compris entre 85 et 95 % du diamètre D2 du deuxième tronçon 42 du logement 26, le diamètre interne minimal étant de préférence sensiblement égal à 90 % du diamètre D2 du deuxième tronçon 42.

En particulier, on a réalisé des essais avec une seringue 100 de 1 ml présentant un corps de seringue 102 de diamètre extérieur égal à 8,15 mm, le diamètre extérieur de la partie médiane 104a étant égal à 4,35 mm, tandis que le diamètre extérieur de la partie tronconique de liaison 104c est égal à 3,85 mm. Pour ce type de seringue, le capuchon 20 utilisé présente alors les dimensions suivantes:
Diamètre du deuxième tronçon 42 du logement 26 (D2) : 4 mm; diamètre du logement 26 au niveau du bourrelet annulaire 70 : 3,6 mm; diamètre de l'ouverture 36 du logement 26 (D1) : 4,7 mm.

Les dimensions qui précèdent sont données pour un capuchon 20 présentant une longueur totale de 23,5 mm pour un diamètre extérieur de 7 mm au niveau de la cinquième portion 62, la quatrième portion 60 allant en se rétrécissant jusqu'à un diamètre extérieur minimal de 6,5 mm.

Bien entendu, les dimensions du capuchon dépendront des dimensions de la seringue, et notamment de sa partie distale portant l'aiguille.

Avec un tel capuchon, on améliore l'étanchéité du logement 26 en autoclave en limitant les risques de déplacement entre le capuchon 20 et la partie distale 104 de la seringue. En effet, le bourrelet annulaire 70 assure un blocage mécanique de la portion distale 104 de la seringue dans le deuxième tronçon 42 du logement 26 pendant le passage en autoclave. En outre, le contact étanche assuré par le deuxième tronçon 42 du logement 26 contre la partie médiane 104a de la portion distale 104 de la seringue engendre une étanchéité microbiologique entre le logement 26 et l'extérieur du dispositif de protection après le passage en autoclave, ce qui garantit une stérilité permanente du logement et de l'aiguille jusqu'à l'utilisation de la seringue 100, plus précisément jusqu'à la séparation entre le capuchon 20 et l'aiguille 106.

Selon l'invention, ledit deuxième tronçon 42 comporte au moins une rainure axiale 420 s'étendant longitudinalement sur une partie de la hauteur dudit deuxième tronçon 42. Comme visible sur les figures, cette au moins une rainure axiale 420 est rectiligne. De préférence, il y a quatre rainures axiales 420 régulièrement angulairement réparties, et disposées en quinconce par rapport aux fentes 70.

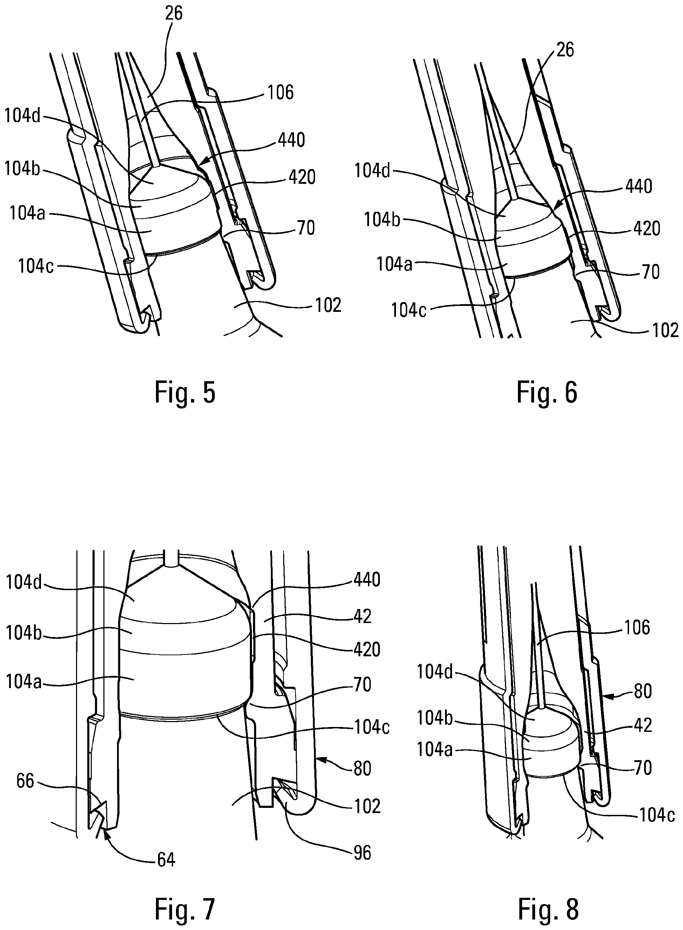

Ces rainures permettent le passage de l'air lors de la stérilisation vapeur, comme schématisé sur la figure 4, ce qui permet d'éviter un gonflement excessif du deuxième tronçon comme illustré sur la figure 8, susceptible de provoquer le désengagement du bourrelet 70 de la seringue. En effet, en l'absence des rainures axiales 420 de l'invention, le gonflement du deuxième tronçon 42 en cas de surpression dans le logement 26 réduit la zone de contact entre le bourrelet 70 et la partie tronconique de liaison 104c, comme visible sur la figure 8. La surpression ayant tendance à déformer radialement le deuxième tronçon 42 vers l'extérieur, le bourrelet 70 peut ne plus suffire pour maintenir le capuchon en position sur la seringue. Avec la présence des rainures 420 de l'invention, ce gonflement est fortement réduit, comme illustré sur la figure 7, et les risques de désengagement sont par conséquent fortement réduits voire éliminés.

Pour garantir l'étanchéité et la stérilité, lesdites rainures axiales 420 ne doivent s'étendre longitudinalement que sur une partie de la hauteur dudit deuxième tronçon 42. Avantageusement, chaque rainure axiale 420 présente au moins une des caractéristiques suivantes: une largeur comprise entre 0,1 et 0,5 mm, une profondeur d'au moins 0,1 mm, une longueur inférieure d'environ 0,5 mm à la longueur dudit deuxième tronçon 42, avantageusement une longueur comprise entre 1,50 et 1,80 mm, de préférence environ 1,65 mm. Bien évidemment, ces dimensions dépendront également des dimensions de la seringue, et notamment de sa partie distale portant l'aiguille.

Avantageusement, ledit troisième tronçon 44 du capuchon comporte une projection radialement saillante 440, de préférence périphérique, adaptée à coopérer avec ladite partie tronconique intermédiaire 104b de la seringue 100 en cas de dépression dans le logement interne 26 du capuchon 20. Ceci permet notamment de limiter la rétractation dudit capuchon 20 sur ladite seringue 100 en cas de dépression dans le logement interne 26 du capuchon, notamment lors de la stérilisation. La figure 6 illustre une telle situation de dépression dans le logement 26, avec le bourrelet 70 qui s'est décalé axialement légèrement de la partie tronconique de liaison 104c, cette rétractation du capuchon étant limitée par la projection radialement saillante 440 qui vient coopérer avec ladite partie tronconique intermédiaire 104b de la seringue.

Avantageusement, ladite projection radialement saillante 440 a une largeur radiale comprise entre 0,1 et 0,5 mm, c'est-à-dire qu'elle s'étend de cette distance radialement vers l'intérieur à partir de la surface interne du troisième tronçon 44. La figure 1, qui compare un capuchon selon l'invention (à gauche) avec un capuchon selon le document EP 1 208 861, montre que la hauteur du deuxième tronçon 42 a été réduite pour prévoir ladite projection radialement saillante 440 à la jonction entre lesdits deuxième et troisième tronçons 42 et 44.

Comme il est illustré notamment sur les figures 2 et 3, la présente invention concerne également un dispositif de protection pour aiguille de seringue qui comporte outre le capuchon élastique 20 présenté précédemment, une coque rigide 80 dans laquelle est logé le capuchon 20.

Ce type de coque 80 est classiquement utilisé pour renforcer la protection de l'utilisateur de la seringue contre une piqûre par aiguille en offrant une protection supplémentaire extérieure rigide qui est difficilement percée par l'aiguille 106.

Cette coque rigide 80 présente une forme générale longitudinale cylindrique de section circulaire, elle est montée de manière coaxiale par rapport au capuchon 20 et elle s'étend entre une extrémité proximale 82 ouverte et une extrémité distale 84 fermée.

La coque rigide 80 est dimensionnée pour permettre l'insertion et le blocage du capuchon 20 à l'intérieur. A cet effet, la cavité 86 délimitée par le contour intérieur de la coque rigide 80 présente une forme qui suit sensiblement la forme extérieure du capuchon 20.

La coque rigide 80 est composée d'une paroi longitudinale 88 qui s'étend depuis l'extrémité proximale 82 jusqu'à l'extrémité distale 84 au niveau de laquelle la paroi longitudinale 88 se prolonge par une paroi terminale 92 qui referme la cavité 86.

La paroi longitudinale 88 peut être munie, entre la paroi terminale 92 et environ deux cinquièmes de la longueur de la paroi longitudinale, de quatre découpes 94 destinées à permettre le passage de la vapeur d'eau sous pression depuis l'enceinte de l'autoclave jusqu'au logement 26.

Ces quatre découpes 94 sont de forme générale longitudinale et elles sont réparties radialement à 90° l'une de l'autre.

Afin de retenir le capuchon 20 à l'intérieur de la cavité 86 de la coque rigide 80, sont prévus des moyens de retenue du capuchon comprenant un rebord rentrant 96, de préférence annulaire, qui forme un élément, de préférence une collerette, faisant saillie vers l'intérieur de la cavité 86.

Ce rebord rentrant 96 est donc logé dans le décrochement formé par la sixième portion 64 du contour extérieur du capuchon 20 de sorte que le capuchon 20 est empêché de sortir de la coque 80 par la venue en butée axiale du rebord rentrant 96 sur la surface d'épaulement 66.

Lors du montage, lorsque le capuchon 20 est enfoncé dans la coque 80 au maximum, la face extérieure essentiellement plane de la paroi d'extrémité 30 du capuchon 20 vient en butée axiale contre la face intérieure de la paroi terminale 92 de la coque 80. Par contre, en position normale, il n'y a pas de contact entre la face extérieure de la paroi d'extrémité 30 du capuchon 20 et la face intérieure de la paroi terminale 92 de la coque 80.

En outre, afin de compléter le blocage du mouvement relatif en translation longitudinale entre le capuchon 20 et la coque rigide 80, plus particulièrement le mouvement du capuchon 20 en direction de la paroi terminale 92 de la coque 80, est prévue, comme moyen de retenue complémentaire, une nervure annulaire 98 disposée sur la face interne de la paroi longitudinale 88 de la coque 80. Cette nervure annulaire 98 est apte à coopérer avec la face extérieure de la paroi latérale 28 du capuchon 20 en venant en butée contre l'épaulement 58 du capuchon 20.

Cet épaulement 58 forme un premier épaulement rentrant en direction de l'extrémité distale 24 du dispositif, ce premier épaulement 58 étant situé sur la face extérieure de la paroi latérale 28 du capuchon 20 en regard du deuxième tronçon 42 du logement 26.

Comme on peut le voir sur la figure 2, la paroi longitudinale 88 comporte, environ à mi-longueur de la coque 80, au moins sur sa face externe, et de préférence (cas de figure illustré) sur toute l'épaisseur de la paroi longitudinale 88 de la coque 80, un épaulement 99 annulaire rentrant en direction de l'extrémité distale 24 du dispositif (le diamètre intérieur de la cavité 86 étant plus faible du côté de l'épaulement 99 situé le plus près de la paroi terminale 92).

Cet épaulement rentrant 99 est formé de façon à se retrouver en face de l'épaulement 54 du capuchon 20 qui forme un deuxième épaulement rentrant en direction de l'extrémité distale du dispositif en étant situé sur la face extérieure de la paroi latérale 28 du capuchon 20 en regard du troisième tronçon 44 du logement 26.

Comme on peut le voir sur la figure 2, l'épaulement rentrant 99 peut se trouver à la fois sur la face interne et sur la face externe de la paroi longitudinale 88 de façon à former un décrochement annulaire de cette paroi.

Cet épaulement 99 annulaire a notamment pour but de faciliter la manipulation du dispositif de protection d'aiguille par les différentes machines de la chaîne de fabrication et de montage.

En outre, il existe, lorsque le capuchon 20 est inséré à l'intérieur de la cavité 86 de la coque 80, un contact entre la cinquième portion 62 du contour extérieur du capuchon 20 et la face intérieure de la paroi longitudinale 88 située entre la nervure 98 et le rebord 96.

Au moment de l'insertion de la portion distale 104 du corps 102 de la seringue dans le logement 26, il y a un écrasement du bourrelet annulaire 70 par la portion distale 104 du corps 102 de la seringue. La paroi latérale 28 du capuchon étant réalisée dans une matière suffisamment souple (par exemple du caoutchouc), une partie de la déformation est absorbée par les fentes 72.

D'autre part, la paroi latérale 28 du capuchon présentant une épaisseur suffisamment mince au regard de la quatrième portion 60 du contour extérieur du capuchon 20, la déformation du bourrelet annulaire 70 engendre un écartement radial de la paroi latérale 28 à cet endroit d'où une déformation de la quatrième portion 60 qui se rapproche de la face intérieure de la paroi longitudinale 88 cylindrique de la coque 80 : ceci est rendu possible grâce à la forme extérieure en tronc de cône de la quatrième portion 60 de la paroi latérale 28 qui se trouve en regard du bourrelet annulaire 70 et à l'espace annulaire disponible sur la face intérieure de la paroi longitudinale 88 de la coque 80 en regard de cette quatrième portion 60.

Il est entendu que la quatrième portion 60 pourrait également présenter une forme de tronc de cône inversée par rapport à celle illustrée, c'est-à-dire une forme allant en se rétrécissant en direction de l'extrémité proximale 22 du capuchon.

Cette quatrième portion 60 pourrait aussi présenter une forme différente de celle d'un tronc de cône, pourvu que cette surface engendre, une fois que le capuchon est logé dans la coque, un espace annulaire libre entre elle et la zone qui lui fait face de la paroi longitudinale 88 de la coque rigide 80. En effet, cet espace annulaire permet de recevoir une partie de la déformation radiale vers l'extérieur de la zone de la paroi latérale 28 du capuchon 20 qui se trouve contre la partie médiane 104a de la portion distale 104 de la seringue.

De manière plus générale, il existe de façon préférentielle un espace, avantageusement annulaire, entre la paroi longitudinale 88 de la coque rigide 80 et la paroi latérale 28 du capuchon 20 qui s'étend de manière longitudinale depuis les découpes 94 jusqu'à la nervure annulaire 98 disposée sur la face interne de la paroi longitudinale 88 de la coque 80, c'est-à-dire jusqu'à l'épaulement saillant 58, de part et d'autre de l'épaulement 99. Cet espace permet le passage de la vapeur d'eau sous pression depuis l'enceinte de l'autoclave jusqu'au logement 26 grâce à la perméabilité aux gaz, notamment à la vapeur d'eau sous pression, du matériau constituant le capuchon 20. De plus, la partie de cet espace qui entoure le deuxième tronçon 42 du logement 26 permet également de recevoir l'expansion radiale de la zone de la paroi latérale 28 du capuchon 20 qui entoure la partie médiane 104a de la portion distale 104 de la seringue.

Ainsi, on comprend que la présence de la coque ne modifie en rien l'utilisation et le fonctionnement du capuchon 20 pour la stérilisation pendant le passage en autoclave et pour le maintien de la stérilité de la seringue 106 après le passage en autoclave. En outre, la présence de la coque ne modifie pas la résistance à l'effort pour séparer le capuchon 20 de la seringue 100, tout en augmentant la sécurité de l'utilisateur contre une piqûre.

Bien que la présente invention ait été décrite en référence à un mode de réalisation, il est entendu que la présente invention n'est pas limitée par celui-ci mais qu'au contraire l'homme du métier peut y apporter toutes modifications utiles sans sortir du cadre de la présente invention tel que défini par les revendications annexées.

## Revendications

1. Dispositif de protection pour aiguille de seringue comportant un capuchon (20) élastique d'aiguille de direction générale longitudinale possédant une extrémité distale (24) fermée et une extrémité proximale (22) ouverte, ledit capuchon (20) comportant une paroi latérale (28), s'étendant depuis ladite extrémité proximale (22) le long d'une zone d'extrémité proximale (32) en délimitant un logement interne (26) destiné à recevoir la portion distale (104) du corps (102) d'une seringue à aiguille (100), et d'une paroi d'extrémité (30) dont l'épaisseur s'étend depuis ladite extrémité distale (24) le long d'une zone d'extrémité distale (34), ladite paroi d'extrémité (30) étant susceptible d'être percée sur une partie de son épaisseur par l'extrémité libre de ladite aiguille (106), ledit logement (26) comprenant, depuis ladite extrémité proximale (22) :
- une ouverture (36) présentant un diamètre maximal (D1),
- un premier tronçon (40) de forme tronconique ou cylindrique de section circulaire,
- un deuxième tronçon (42) cylindrique de section circulaire présentant un diamètre (D2) inférieur audit diamètre maximal (D1) et destiné à loger la portion distale (104) du corps de seringue (102) qui porte ladite aiguille (106),
- un troisième tronçon (44) qui va en se rétrécissant depuis ledit deuxième tronçon (42) en direction du fond (38) dudit logement (26), ladite paroi latérale (28) étant en outre munie d'un bourrelet annulaire (70) disposé dans ledit logement (26) entre lesdits premier et deuxième tronçons (40, 42) dudit logement (26), au moins une fente (72) s'étendant en direction longitudinale sur ledit bourrelet annulaire (70),
**caractérisé en ce que** ledit deuxième tronçon (42) comporte au moins une rainure axiale rectiligne (420) s'étendant longitudinalement sur une partie de la hauteur dudit deuxième tronçon (42).

2. Dispositif selon la revendication 1, dans lequel une pluralité, notamment quatre, fentes (72) régulièrement angulairement réparties s'étendent en direction longitudinale sur ledit bourrelet annulaire (70), et une pluralité, notamment quatre, rainures (420) régulièrement angulairement réparties s'étendent en direction longitudinale sur ledit deuxième tronçon (42).

3. Dispositif selon la revendication 2, dans lequel lesdites fentes (72) sont disposées en quinconce avec lesdites rainures (420).

4. Dispositif selon l'une quelconque des revendications précédentes, dans lequel ladite au moins une rainure a une largeur comprise entre 0,1 et 0,5 mm et/ou une profondeur d'au moins 0,1 mm et/ou une longueur inférieure d'environ 0,5 mm à la longueur dudit deuxième tronçon (42), avantageusement une longueur comprise entre 1,50 et 1,80 mm, de préférence environ 1,65 mm.

5. Dispositif selon l'une quelconque des revendications précédentes, dans lequel ledit troisième tronçon (44) comporte une projection radialement saillante (440), de préférence périphérique, adaptée à coopérer avec ladite partie tronconique intermédiaire (104b) de la seringue (100) en cas de dépression dans le logement interne (26) du capuchon (20) pour éviter une rétractation dudit capuchon (20) sur ladite seringue (100).

6. Dispositif selon la revendication 5, dans lequel ladite projection radialement saillante (440) a une largeur radiale comprise entre 0,1 et 0,5 mm.

7. Dispositif selon l'une quelconque des revendications précédentes, comportant en outre une coque rigide (80) de forme générale longitudinale et cylindrique de section circulaire présentant une extrémité proximale (82) ouverte et une extrémité distale (84) au moins partiellement fermée, ladite coque (80) comprenant une paroi longitudinale (88) s'étendant depuis ladite extrémité proximale (82) jusqu'à ladite extrémité distale (84) et une paroi terminale (92) située à son extrémité distale, ladite coque (80) étant destinée à entourer en le contenant ledit capuchon (20) élastique d'aiguille et étant pourvue de moyens de retenue (96, 98, 99) dudit capuchon (20).

8. Dispositif selon la revendication 7, dans lequel lesdits moyens de retenue comportent un rebord annulaire rentrant (96) formant l'extrémité libre proximale de la coque (80) et présentant un diamètre intérieur apte à retenir dans la coque (80) l'extrémité proximale de la paroi latérale (28) du capuchon (20).

9. Dispositif selon la revendication 8, dans lequel l'extrémité proximale de la surface extérieure de la paroi latérale (28) du capuchon (20) comporte un épaulement annulaire rentrant (64) en direction de l'extrémité proximale (22) du dispositif et apte à coopérer avec ledit rebord annulaire (96).

10. Dispositif selon l'une quelconque des revendication 7 à 9, dans lequel lesdits moyens de retenue comportent en outre une nervure annulaire (98) disposée sur la face interne de la paroi longitudinale (88) de la coque (80) et apte à coopérer avec un premier épaulement rentrant (58) en direction de l'extrémité distale (24) du dispositif et situé sur la surface extérieure de la paroi latérale (28) du capuchon (20) en regard du deuxième tronçon (42) dudit logement (26).

## Patentansprüche

1. Schutzvorrichtung für eine Spritzennadel, aufweisend eine elastische Nadelkappe (20) in allgemeiner Längsrichtung mit einem geschlossenen distalen Ende (24) und einem offenen proximalen Ende (22), wobei die Kappe (20) eine Seitenwand (28), die sich von dem proximalen Ende (22) entlang eines proximalen Endbereichs (32) erstreckt und dabei eine interne Aufnahme (26) begrenzt, die dafür ausgelegt ist, den distalen Abschnitt (104) des Körpers (102) einer Spritzennadel (100) aufzunehmen, und eine Endwand (30) aufweist, deren Dicke sich von dem distalen Ende (24) entlang eines distalen Endbereichs (34) erstreckt, wobei die Endwand (30) dafür geeignet ist, auf einem Teil ihrer Dicke von dem freien Ende der Nadel (106) durchdrungen zu werden, wobei die Aufnahme (26) von dem proximalen Ende (22) aus aufweist:
- eine Öffnung (36), die einen maximalen Durchmesser (D1) hat,
- einen ersten Teilabschnitt (40) von kegelstumpfartiger oder zylindrischer Form mit kreisförmigem Querschnitt,
- einen zweiten zylindrischen Teilabschnitt (42) mit kreisförmigem Querschnitt, der einen Durchmesser (D2) hat, der kleiner ist als der maximale Durchmesser (D1) und der dafür ausgelegt ist, den distalen Abschnitt (104) des Spritzenkörpers (102) aufzunehmen, der die Nadel (106) trägt,
- einen dritten Teilabschnitt (44), der sich von dem zweiten Teilabschnitt (42) in Richtung zum Boden (38) der Aufnahme (26) verengt, wobei die Seitenwand (28) des Weiteren mit einem ringförmigen Wulst (70) versehen ist, der in der Aufnahme (26) zwischen dem ersten und dem zweiten Teilabschnitt (40, 42) der Aufnahme (26) angeordnet ist, wobei sich mindestens ein Schlitz (72) in Längsrichtung auf dem ringförmigen Wulst (70) erstreckt,
**dadurch gekennzeichnet, dass** der zweite Teilabschnitt (42) mindestens eine axiale, geradlinige Nut (420) umfasst, die sich längs über einen Teil der Höhe des zweiten Teilabschnitts (42) erstreckt.

2. Vorrichtung nach Anspruch 1, wobei sich mehrere, insbesondere vier, Schlitze (72), die winkelig gleichmäßig verteilt sind, in Längsrichtung auf dem ringförmigen Wulst (70) erstrecken, und sich mehrere, insbesondere vier, Nuten (420), die winkelig gleichmäßig verteilt sind, in Längsrichtung auf dem zweiten Teilabschnitt (42) erstrecken.

3. Vorrichtung nach Anspruch 2, wobei die Schlitze (72) in Fünfpunktanordnung mit den Nuten (420) angeordnet sind.

4. Vorrichtung nach einem der vorhergehenden Ansprüche, wobei die mindestens eine Nut eine Breite zwischen 0,1 und 0,5 mm und/oder eine Tiefe von mindestens 0,1 mm und/oder eine Länge von weniger als etwa 0,5 mm als die Länge des zweiten Teilabschnitts (42), vorteilhafterweise eine Länge zwischen 1,50 und 1,80 mm, vorzugsweise etwa 1,65 mm aufweist.

5. Vorrichtung nach einem der vorhergehenden Ansprüche, wobei der dritte Teilabschnitt (44) einen vorzugsweise umfänglichen, radial vorstehenden Vorsprung (440) aufweist, der dazu geeignet ist, bei einem Herunterdrücken in die interne Aufnahme (26) der Kappe (20) mit dem kegelstumpfartigen Zwischenteil (104b) der Spritze (100) zusammenzuwirken, um ein Zurückziehen der Kappe (20) auf der Spritze (100) zu verhindern.

6. Vorrichtung nach Anspruch 5, wobei der radial vorstehende Vorsprung (440) eine radiale Breite zwischen 0,1 und 0,5 mm aufweist.

7. Vorrichtung nach einem der vorhergehenden Ansprüche, des Weiteren aufweisend eine starre Schale (80) von allgemeiner länglicher und zylindrischer Form mit kreisförmigem Querschnitt, aufweisend ein offenes proximales Ende (82) und ein distales Ende (84), das zumindest teilweise geschlossen ist, wobei die Schale (80) eine Längswand (88), die sich von dem proximalen Ende (82) bis zum distalen Ende (84) erstreckt, und eine Endwand (92) aufweist, die sich an ihrem distalen Ende befindet, wobei die Schale (80) dazu ausgelegt ist, die elastische Nadelkappe (20) zu umgeben und dabei aufzunehmen, und mit Haltemitteln (96, 98, 99) der Kappe (20) versehen ist.

8. Vorrichtung nach Anspruch 7, wobei die Haltemittel einen ringförmigen, eingezogenen Umschlag (96) aufweisen, der das freie proximale Ende der Schale (80) bildet und einen Innendurchmesser hat, der dafür geeignet ist, das proximale Ende der Seitenwand (28) der Kappe (20) in der Schale (80) zu halten.

9. Vorrichtung nach Anspruch 8, wobei das proximale Ende der Außenfläche der Seitenwand (28) der Kappe (20) einen ringförmigen, eingezogenen Absatz (64) in Richtung des proximalen Endes (22) der Vorrichtung aufweist und in der Lage ist, mit dem ringförmigen Umschlag (96) zusammenzuwirken.

10. Vorrichtung nach einem der Ansprüche 7 bis 9, wobei die Haltemittel des Weiteren eine ringförmige Rippe (98) aufweisen, die auf der Innenseite der Längswand (88) der Schale (80) angeordnet und in der Lage ist, mit einem ersten eingezogenen Absatz (58) in Richtung des distalen Endes (24) der Vorrichtung zusammenzuwirken, der sich auf der Außenfläche der Seitenwand (28) der Kappe (20) in Bezug auf den zweiten Teilabschnitt (42) der Aufnahme (26) befindet.

## Claims

1. A protection device for protecting a syringe needle, the device comprising an elastomeric needle cap (20) that extends in a generally longitudinal direction and that has a closed distal end (24) and an open proximal end (22), said cap (20) comprising a lateral wall (28) that extends from said proximal end (22) along a proximal end zone (32), defining an internal housing (26) for receiving the distal portion (104) of the body (102) of a needle syringe (100), and an end wall (30) having a thickness that extends from said distal end (24) along a distal end zone (34), said end wall (30) being suitable for being perforated over a fraction of its thickness by the free end of said needle (106), said housing (26) comprising, from said proximal end (22):
• an opening (36) that presents a maximum diameter (D1);
• a first segment (40) of frustoconical or circularly cylindrical shape;
• a circularly-cylindrical second segment (42) that presents a diameter (D2) that is less than said maximum diameter (D1), and that is for housing the distal portion (104) of the syringe body (102) that carries said needle (106); and
• a third segment (44) that tapers from said second segment (42) towards the end wall (38) of said housing (26), said lateral wall (28) also being provided with an annular bead (70) that is arranged in said housing (26) between said first and second segments (40, 42) of said housing (26), at least one slot (72) extending in a longitudinal direction across said annular bead (70);
the device being **characterized in that** said second segment (42) includes at least one rectilinear axial groove (420) that extends longitudinally over a fraction of the height of said second segment (42).

2. A device according to claim 1, wherein a plurality of slots (72), in particular four slots, angularly distributed in regular manner, extend in a longitudinal direction across said bead (70), and a plurality of grooves (420), in particular four grooves, angularly distributed in regular manner, extend in a longitudinal direction across said second segment (42).

3. A device according to claim 2, wherein said slots (72) are arranged in a staggered configuration with said grooves (420).

4. A device according to any preceding claim, wherein said at least one groove has a width lying in the range 0.1 mm to 0.5 mm and/or a depth of at least 0.1 mm and/or a length that is about 0.5 mm shorter than the length of said second segment (42), advantageously a length lying in the range 1.50 mm to 1.80 mm, preferably about 1.65 mm.

5. A device according to any preceding claim, wherein said third segment (44) includes a preferably-peripheral radially-projecting projection (440) that is adapted to co-operate with said frustoconical intermediate portion (104b) of the syringe (100) in the event of suction in the internal housing (26) of the cap (20), so as to avoid said cap (20) shrinking onto said syringe (100).

6. A device according to claim 5, wherein said radially-projecting projection (440) has a radial width lying in the range 0.1 mm to 0.5 mm.

7. A device according to any preceding claim, further including a rigid shell (80) of generally longitudinal and of circularly cylindrical shape, presenting a proximal end (82) that is open and a distal end (84) that is closed at least in part, said shell (80) comprising a longitudinal wall (88) that extends from said proximal end (82) to said distal end (84) and an end wall (92) that is situated at its distal end, said shell (80) being for surrounding and containing said elastomeric needle cap (20) and being provided with retaining means (96, 98, 99) for retaining said cap (20).

8. A device according to claim 7, wherein said retaining means include a re-entrant rim (96) that forms the proximal free end of the shell (80) and that presents an inside diameter that is suitable for retaining, in the shell (80), the proximal end of the lateral wall (28) of the cap (20).

9. A device according to claim 8, wherein the proximal end of the outside surface of the lateral wall (28) of the cap (20) includes an annular shoulder (64) that is re-entrant towards the proximal end (22) of the device and that is suitable for co-operating with said annular rim (96).

10. A device according to any one of claims 7 to 9, wherein said retaining means further include an annular rib (98) that is arranged on the inside face of the longitudinal wall (88) of the shell (80) and that is suitable for co-operating with a first shoulder (58) that is re-entrant towards the distal end (24) of the device and that is situated on the outside surface of the lateral wall (28) of the cap (20) level with the second segment (42) of said housing (26).
